(19) Europäisches Patentamt
European Patent Office
Office européen des brevets

(11) Publication number : **0 468 634 A1**

(12) **EUROPEAN PATENT APPLICATION**

(21) Application number : **91305484.7**

(51) Int. Cl.$^5$ : **A61K 49/00**

(22) Date of filing : **18.06.91**

(30) Priority : **18.06.90 US 540429**

(43) Date of publication of application :
**29.01.92 Bulletin 92/05**

(84) Designated Contracting States :
**AT BE CH DE DK ES FR GB GR IT LI LU NL SE**

(71) Applicant : **THE DOW CHEMICAL COMPANY**
**2030 Dow Center Abbott Road**
**Midland, MI 48640 (US)**

(72) Inventor : **Simon, Jaime**
**Route 1, Box 120-G**
**Angleton, Texas 77515 (US)**
Inventor : **Garlich, Joseph R.**
**301 Southern Oak Drive**
**Lake Jackson, Texas 77566 (US)**

(74) Representative : **Raynor, John et al**
**W.H. Beck, Greener & Co 7 Stone Buildings**
**Lincoln's Inn**
**London WC2A 3SZ (GB)**

(54) **The use of macrocyclic aminophosphonic acid complexes as imaging agents.**

(57) A method for diagnostic use in an MRI agent is disclosed wherein the composition comprises a complex of a macrocyclic aminophosphonic acid, preferably 1,4,7,10-tetraazacyclododecane-1,4,7,10-tetramethylenephosphonic acid, and a paramagnetic nuclide, such as $Gd^{+3}$, $Mn^{+2}$, $Mn^{+3}$ or $Fe^{+3}$. The composition is bone seeking and stable over a wide pH range.



Jouve, 18, rue Saint-Denis, 75001 PARIS

The present invention is directed to the use of macrocyclic aminophosphonic acid complexes as imaging agents.

Complexes of paramagnetic metal ions have received considerable attention over the last five years, particularly with respect to their ability to enhance the image obtained from magnetic resonance imaging (MRI) scans of patients. The primary chelants of interest for this use have been aminocarboxylate analogs such as diethylenetriaminepentaacetic acid (DTPA), which is currently the only commercial MRI pharmaceutical available in the United States (trade name MAGNEVIST™ by Schering). The primary metals of interest for MRI uses are paramagnetic ions of gadolinium ($Gd^{+3}$), iron ($Fe^{+3}$) and manganese ($Mn^{+2}$ or $Mn^{+}3$). These ions effectively reduce the relaxation rates of water by virtue of the strong dipolar interactions with the chelated metal.

For a paramagnetic chelate to be of use as an MRI contrast agent it must alter the relaxation rate of water in various tissue, be kinetically inert, and clear the body rapidly (within hours of injection). Recently, 1,4,7,10-tetraazacyclododecane-1,4,7,10-tetraacetic acid (DOTA) was introduced in Europe (trade name DOTAREM™ by Guerbet) as an effective Gd chelate for MRI applications; however, the compound is not yet available in the United States. Discussions of Gd-DOTA complexes are found in U. S. Patents 4,877,600 and 4,885,363.

Recently, it has been found that Gd chelates of 1,4,7,10-tetraazacyclododecane-1,4,7,10-tetramethylenephosphonic acid (DOTMP) behave similarly to DOTA with respect to the necessary features of an MRI contrast agent. Chelates of DOTMP display a high degree of kinetic inertness, have very favorable biodistributions, and enhance the relaxivity of water.

Furthermore, published European Application 375,376 discloses the use of the ligands of the present invention as complexes with radioactive nuclides of the lanthanide series for the treatment of calcific tumors and relief of bone pain. The complexes have shown a preference to attach to calcific sites, e.g., bone, metastatic and primary bone tumor, and calcified soft tissue. Calcified soft tissue can be a result of a heart attack where the cardiac tissue that is damaged is calcified. Also these complexes can deliver the radionuclide to the calcific surface without the need for excess ligand. Thus a lower dose of the complex is achievable and a lower metal to ligand ratio is also possible.

The present invention concerns macrocyclic aminophosphonic acid complexes for use as magnetic resonance imaging (MRI) agents of animals and compositions and formulations having as their active ingredient a paramagnetic nuclide complexed with a macrocyclic aminophosphonic acid.

Specifically, the present invention concerns a method of obtaining a magnetic resonance image of an animal which comprises administering to the animal a calcific site seeking composition comprising a complex between:-

(1) a macrocyclic aminophosphonic acid of the formula

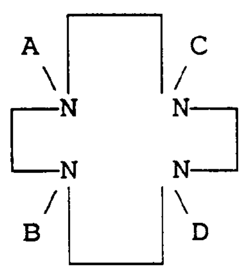

(I)

wherein A, B, C and D are each independently hydrogen, or hydrocarbyl group having from 1 to 8 carbon atoms, or a moiety of the formula:-

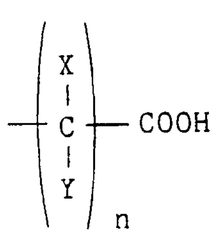 , 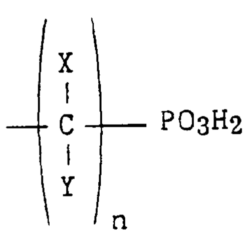 , or 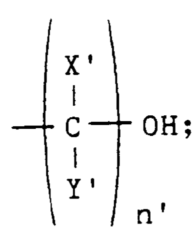

or a physiologically acceptable salt of such a moiety wherein: each X and each Y independently is hydrogen or a hydroxyl, carboxyl, phosphonic or hydrocarbyl group having from 1 to 8 carbon atoms; each X′ and each

Y′ independently is hydrogen, a methyl or an ethyl group; n′ is 2 or 3, with the proviso that at least two of said nitrogen substituents is A, B, C and D is a phosphorus-containing group; and (2) at least one paramagnetic nuclide; wherein the molar ratio of (1) to (2) is at least about 1:1.

The compositions can be used in a lower dose and/or a lower ligand to metal ratio than known calcific site seeking MRI agents. The present compositions can deliver the paramagnetic nuclide specifically to calcific surfaces at or about a stoichiometric ratio of ligand to metal ion. The target specificity feature makes a DOTMP complex particularly attractive as an MRI contrast agent, because this complex would create a high contrast image of bone or any region in the body where calcified tissue resides. For example, heart imaging where calcification is known to occur following a heart attack.

In addition the present invention also includes formulations for use in the method having at least one of the paramagnetic nuclide(s) complexed with at least one of the macrocyclic aminophosphonic acids of formula (I) and a pharmaceutlcally-acceptable carrier, excipient or vehicle therefor.

The process for preparing such formulations are well known. The formulations are sterile and may be in the form of a suspension, injectable solution or other suitable pharmaceutically acceptable formulations. Pharmaceutically acceptable suspending media, with or without adjuvants, may be used.

The present invention includes the use of the complex, composition or formulation described herein in combination with one or more other agents, which also function as MRI agents.

Surprisingly, it has now been found that macrocyclic aminophosphonic acid complexes, for example Gd-DOTMP, have excellent calcific site seeking abilities coupled with low soft tissue localization *in vivo* at low ligand to metal molar ratios, and even at 1:1 ligand to metal molar metal ratios. This is in contrast to other Gd complexes of aminophosphonates, such as Gd-EDTMP which are described by I. K. Adzamli et al. in *J. Med. Chem*, 32, 139-144 (1989). The unexpected inertness of the DOTMP complexes with rare earths has been demonstrated with Sm-DOTMP (see published European Application 375,376) and we know to be stable from pH 1 to pH 13 at a 1:1 ligand to metal molar ratio. In contrast, we have found with Sm-EDTMP, even at a 7:1 ligand to metal molar ratio, that Sm-EDTMP is stable only around pH 7 and will readily become uncomplexed at lower and higher pH. Thus, by analogy the other rare earth metal ions, such as Gd, should behave similarly.

While not wishing to be bound by theory, it is believed that the advantageous results of the present invention are obtained because of the excellent target to nontarget tissue ratios *in vivo* for the Gd-DOTMP complex which is a result of the degree of the inertness and which stability for the complex of DOTMP with Gd metal ion.

Specifically, this invention concerns a method of magnetic imaging of an animal which comprises administering to the animal a calcific site seeking composition of a complex having a compound of formula (I) and a paramagnetic nuclide as defined hereinabove. The results of such imaging may be utilised in subsequent diagnosis.

The preferred macrocyclic aminophosphonic acid is 1,4,7,10-tetraazacyclododecane-1,4,7,10-tetramethylenephosphonic acid (DOTMP). The composition can be administered as a formulation with suitable pharmaceutically-acceptable carriers.

A "paramagnetic nuclide" of this invention means a metal ion which displays spin angular momentum and/or orbital angular momentum. The two types of momentum combine to give the observed paramagnetic moment in a manner that depends largely on the atoms bearing the unpaired electron and, to a lesser extent, upon the environment of such atoms. The paramagnetic nuclides found to be useful in the practice of the invention are gadolinium ($Gd^{+}3$), iron ($Fe^{+}3$) and manganese ($Mn^{+2}$ or $Mn^{+}3$), with $Gd^{+}3$ being preferred.

The paramagnetic nuclide and ligand may be combined under any conditions which allow the two to form a complex. Generally, mixing in water at a controlled pH (the choice of pH is dependent upon the choice of ligand and paramagnetic nuclide) is all that is required. The complex formed is by a chemical bond and results in a relatively stable paramagnetic nuclide composition, e.g. stable to the disassociation of the paramagnetic nuclide from the ligand.

The compositions of the present invention can be used in a lower dose and/or a lower ligand to metal ratio than known calcific site seeking MRI agents. The present compositions can deliver the paramagnetic nuclide(s) specifically to calcific surfaces at or about a stoichiometric ratio of ligand to metal ion. One advantage to this stoichiometric ratio that is provided by the present compositions is that it allows delivery of larger amounts of metal to the target calcific site.

The macrocyclic aminophosphonic acid complexes when administered at a ligand to metal molar ratio of at least about 1:1, preferably from 1:1 to 3:1, more preferably from 1:1 to 1.5:1, give biodistributions that are consistent with excellent skeletal agents. By contrast, certain other nonmacrocyclic aminophosphonic acid complexes result in some localization in soft tissue (e.g. liver) if excess amounts of ligand are not used. A large excess of ligand is undesirable since uncomplexed ligand may be toxic to the animal or may result in cardiac arrest or hypocalcemic convulsions. In addition, the macrocyclic aminophosphonic acid ligands are useful when large amounts of metal are required. In this case, the macrocyclic aminophosphonic acid ligands have the ability

to deposit larger amounts of metal in the bone than is possible when using the same amount of non-cyclic aminophosphonic acid ligands.

Aminophosphonic acids can be prepared by a number of known synthetic techniques. Of particular importance is the reaction of a compound containing at least one reactive amine hydrogen with a carbonyl compound (aldehyde or ketone) and phosphorous acid or derivative thereof. The amine precursor (1,4,7,10-tetraazacyclododecane) employed in making the macrocyclic aminophosphonic acids is a commercially available material.

Methods for carboxyalkylating to give amine derivatives containing a carboxyalkyl group are well known (U.S. Patent 3,726,912) as are the methods which give alkyl phosphonic and hydroxyalkyl (U.S. Patent 3,398,198) substituents on the amine nitrogens.

The ligands of this invention have been prepared according to the procedure discribed in our published European Application 375,376.

The compositions and formulations of the present invention are suitable for use as MRI contrast agents, especially for calcific sites.

In addition, the present invention also includes formulations having at least one of the paramagnetic nuclide(s) complexed with at least one of the macrocyclic aminophosphonic acids as defined above, especially those macrocyclic aminophosphonic acids of Formula (II), and a pharmaceutically-acceptable carrier, excipient or vehicle therefor. The methods for preparing such formulations are well known. The formulations are sterile and may be in the form of a suspension, injectable solution or other suitable pharmaceutically-acceptable formulations. Pharmaceutically-acceptable suspending media, with or without adjuvants, may be used. The sterile compositions are suitable for administration to an animal.

For the purpose of convenience, the compositions having a paramagnetic nuclide-macrocyclic aminophosphonic acid complex of the present invention will frequently be referred to herein as "paramagnetic nuclide compositions" or "compositions" and the macrocyclic aminophosphonic acid derivative referred to as the "ligand" or "chelant".

As used herein, the term "animals" means warm blooded mammals, including humans, and is meant to encompass animals in need ofimaging for diagnostic purposes.

For the purpose of the present invention, the complexes described herein and physiologically-acceptable salts thereof are considered equivalent in the compositions. Physiologically-acceptable salts refer to the acid addition salts of those bases which will form a salt with at least one acid group of the ligand or ligands employed and which will not cause a significant adverse physiological effect when administered to an animal at dosages consistent with good pharmacological practice. Suitable bases include, for example, the alkali metal and alkaline earth metal hydroxides, carbonates, and bicarbonates such as sodium hydroxide, potassium hydroxide, calcium hydroxide, potassium carbonate, sodium bicarbonate, magnesium carbonate and the like, ammonia, primary, secondary and tertiary amines and the like. Physiologically acceptable salts may be prepared by treating the macrocyclic aminophosphonic acid as defined above, especially those of Formula (II), with an appropriate base.

The formulations of the present invention are in the solid or liquid form containing the paramagnetic nuclide complexed with the ligand. These formulations may be in kit form such that the two components are mixed at the appropriate time prior to use. Whether premixed or as a kit, the formulations usually require a pharmaceutically-acceptable carrier. Frequently, it is desirable to have a buffer present in the formulation. Examples of suitable buffers are phosphate, bicarbonate and N-2-hydroxyethylpiperazine-N′-2-ethanesulfonic acid (HEPES).

Injectable compositions of the present invention may be either in suspension or solution form. In the preparation of suitable formulations it will be recognized that, in general, the water solubility of the salt is greater than the free acid. In solution form the complex (or when desired the separate components) is dissolved in a pharmaceutically acceptable carrier. Such carriers comprise a suitable solvent, preservatives such as benzyl alcohol, if needed, and buffers. Useful solvents include, for example, water, aqueous alcohols, glycols, and phosphonate or carbonate esters. Such aqueous solutions contain no more than 50% of the organic solvent by volume.

Injectable suspensions as compositions of the present invention require a liquid suspending medium, with or without adjuvants, as a carrier. The suspending medium can be, for example, aqueous polyvinyl-pyrrolidone, inert oils such as vegetable oils or highly refined mineral oils, or aqueous carboxymethlycellulose. Suitable physiologically acceptable adjuvants, if necessary to keep the complex in suspension, may be chosen from among thickeners such as carboxymethyl-cellulose, polyvinylpyrrolidone, gelatin, and the alginates. Many surfactants are also useful as suspending agents, for example, lecithin, alkylphenol, polyethylene oxide adducts, naphthalenesulfonates, alkylbenzenesulfonates, and the polyoxyethylene sorbitan esters. Many substances which effect the hydriphibicity, density, and surface tension of the liquid suspension medium can assist in making injectable suspensions in individual cases. For example, silicone antifoams, sorbitol and sugars are all useful suspending agents.

Combinations of the various above noted paramagnetic nuclides can be administered as MRI agents, either sequentially or concurrently, if desired to obtain the contrast or diagnostic information desired. The use of different paramagnetic nuclides complexed with one of the ligands of the present could desirably provide confirming diagnostic information. The combinations can be complexed as herein described by complexing them simultaneously, mixing two separately complexed paramagnetic nuclides, or administering two different complexed paramagnetic nuclides sequentially. The comnosition or formulation may be administered as a single dose or as multiple doses over a longer period of time.

The compositions of the present invention may be used in conjunction with other active agents and/or ingredients that enhance the diagnosticeffectiveness of the compositions and/or facilitate easier administration of the compositions.

Studies to determine the qualitative biodistribution of the various complexes of the invention were conducted by injecting into rats compositions according to the invention containing $^{159}Gd$ as a radioactive tracer, and obtaining the gamma ray images of the entire animal at various times up to two hours after injection.

Quantitative biodistributions were obtained by injecting 50-100 microliters of the composition into the rail vein of unanesthetized male Sprague Dawley rats. The rats were then placed in cages lined with absorbent paper in order to collect all urine excreted prior to sacrifice. After a given period of time, the rats were sacrificed by cervical dislocation and the various tissues dissected. The samples were then rinsed with saline, blotted dry on absorbent paper and weighed. The radioactivity in the samples was measured with a NaI scintillation counter.

The invention will be further clarified by a consideration of the following examples, which are intended to be purely exemplary of the present invention.

STARTING MATERIALS

Example A: Preparation of DOTMP

In a 100-mL three necked round-bottomed flask equipped with a thermometer, reflux condenser, and heating mantle was added 3.48 g (20.2 mMol) of 1,4,7,10-tetraazacyclododecane and 14 mL of water. This solution was treated with 17.2 mL of concentrated HCl and 7.2 g of $H_3PO_3$ (87.8 mMol) and heated to 105°C. The refluxing suspension was stirred vigorously and treated dropwise with 13 g (160.2 mMol) of formaldehyde (37 wt% in water) over a one hour period. At the end of this time the reaction was heated at reflux an additional 2 hours after which the heat was removed and the reaction solution allowed to cool and set at room temperature for 62.5 hours. The reaction solution was then concentrated *in vacuo* at 40°C to a viscous reddish brown semisolid. A 30 mL portion of water was added to the semisolid which started to dissolve but then began to solidify. The whole suspension was then poured into 400 mL of acetone with vigorously stirring. The resulting off-white precipitate was vacuum filtered and dried overnight to give 10.69 g (97% yield) of crude DOTMP. A 2.0 g (3.65 mMol) sample of the crude DOTMP was dissolved in 2 mL of water by the addition of 700 μL of concentrated ammonium hydroxide (10.0 mMol) in 100 μL portions to give a solution at pH of 2-3. This solution was then added all at once to 4.5 mL of 3N HCl (13.5 mMol), mixed well, and allowed to set. Within one hour small squarish crystals had begun to form on the sides of the glass below the surface of the liquid. The crystal growth was allowed to continue undisturbed for an additional 111 hours after which time the crystals were gently bumped off of the vessel walls, filtered, washed with 3 mL portions of water, four times, and air dried to constant weight to give 1.19 g (60% yield) of white crystalline solid DOTMP.

Example B: Preparation of DOTMP

A 250 mL three-necked, round-bottomed flask was loaded with 6.96 g (0.04 moles) of 1,4,7,10-tetraazacyclododecane. To this flask was added 14.5 g (0.177 moles) of phosphorous acid, 30 mL of deionized water and 28 mL of concentrated hydrochloric acid (0.336 moles).

The flask was attached to a reflux condenser and fitted with a stir bar, and a thermometer adapted with a thermowatch controller. A separate solution of 26.0 g (0.32 moles) of aqueous 37% formaldehyde solution was added to a 100 mL addition funnel and attached to the flask. The flask was brought to reflux temperature (about 105°C) with vigorous stirring. The formaldehyde solution was added dropwise over a 30-40 minute interval. The solution was heated and stirred for an additional three hours then cooled slowly to ambient temperature.

The reaction solution was transferred to a 500 mL round-bottomed flask and attached to a rotary evaporation apparatus. The solution was taken down to a viscous, amber semi-solid (note - temperature never exceeded 40°C). This semi-solid was treated with approximately 300 mL of HPLC grade acetone producing a light brown, sticky viscous oil. This oil was dissolved in 22 mL of water and added slowly with vigorous stirring

to IL of acetone. The acetone was decanted and the light colored oil dried under vacuum to give 16.6 g (76% yield) of crude DOTMP. To 13.1 g of this crude DOTMP was added 39.3 g of deionized water along with a seed crystal and the solution allowed to stand overnight. The resulting precipitate was vacuum filtered, washed with cold water, and dried under vacuum to give 4.75 g of DOTMP (36% yield).

A further purification was performed by dissolving 3.0 g (5.47 mMol) of DOTMP from above in 3 mL of water by the addition of 2.2 mL (31.5 mMol) of concentrated ammonium hydroxide. This solution was made acidic by the addition of 2.4 mL (28.8 mMol) of concentrated HCl at which time a white solid precipitated. This precipitate was vacuum filtered and dried to give 2.42 g (81% yield) of purified DOTMP characterized by a singlet at 11.5 ppm (relative to 85% $H_3PO_4$) in the $^{31}P$ decoupled NMR spectrum.

Example C: Preparation of DOTMP

A 50 mL three-necked, round-bottomed flask was loaded with 2.0 g (11.6 mMol) of 1,4,7,10-tetraazadodecane. The amine was made soluble with the addition of 5.56 g of concentrated HCl and 3.85 g of phosphorous acid. The flask was fitted with a thermometer and reflux condenser, then placed on a heater/stirrer. With constant stirring, the solution was brought up to reflux temperature. A Sage™ syringe pump was loader with 4.33 g of 37% (vol/vol) formaldehyde solution. The solution was slowly pumper into the reaction flask at a rate of 0.1 mL/min flow. The reaction was heated at this temperature for an additional 6 hrs. The reaction solution was then cooled, filtered through a medium glass fritted filter funnel, and rotaevaporated to dryness. The solid was washed twice with deionized water, then rotaevaporated to a viscous, amber semisolid. The product was triturated in methanol and filtered to provide 1.6 g of a tan solid. The methanol layer was taken to dryness (a dark amber viscous liquid) and dried overnight in a vacuum oven. Total weight of product was 6.17 g.

The two above fractions were placed in a 100 mL round-bottomed flask and the phosphonomethylation process run again overnight. The solution was cooled and worked up as before. The total amount of product was 4.3 g. A $^{31}P$ NMR of the product was consistent with that of DOTMP.

FINAL PRODUCTS

Example 1: Preparation of Gd-DOTMP

A flask was tared and loaded with 200 mg (0.36 mMol) of DOTMP, prepared by the procedure of Example C, and 3.0 mL of deionized water. Concentrated $NH_4OH$ was added to the flask in microliter increments until all solid was soluble. The solution was then heated to 80°C with stirring. To the solution was added dropwise with stirring 1.5 mL of a 0.2M solution of gadolinium-triacetate (0.300 mMol). The resulting solution was maintained at 80°C for 10 min. The solution was cooled and the pH was adjusted to 7.0 using 0.5 mL of 6N HCl. To 100 µL of the complex was added, for dilution, 1.0 mL of $D_2O$ and 3.9 mL of deionized water to give a final complex concentration of 680 µMolar. This solution contains 20% of $D_2O$ (vol/vol).

Example 2: Determination of $T_1$ Relaxation Time

The complex from Example 1 was deoxygenated by bubbling dry nitrogen gas through it for 20 min. The sample was then placed in a 5 mm NMR tube and placed in a 270 MHz spectrometer for determination of the water proton relaxation time.

The NMR experiment used to measure the relaxation time was the standard inversion recovery technique for measuring spin-lattice ($T_1$) relaxation times. In this technique the intensity of the water proton NMR resonance is measured as a function of a variable delay time between radiofrequency pulses during which the spin system relaxes toward equilibrium. The final data, which consisted of peak intensities versus delay time, was plotted and fitted to an exponential function. The characteristic time for spin magnetization to reestablish itself ($T_1$) is obtained from the time constant of the fitted exponential function. The value for $T_1$ for the complex of Example 1 was 244 mS. This compares favorably to a value of 422 mS for a Gd-DTPA, and 540 mS for Gd-DOTA when the experiment was repeated using these complexes. (The Gd-DTPA and Gd-DOTA are known commercial MRI agents.)

Example 3: Biodistribution of $^{159}Gd$-DOTMP

A 1.45 mg sample of DOTMP, prepared by the procedure of Example B, was placed in a vial and dissolved in 760 µL of water and 5 µL of 50% (wt/wt) of NaOH. A volume of 1000 µL of Gd solution (0.3 mM Gd in O.1N HCl), which contained tracer quantities of $^{159}Gd$, was placed in a separate vial and 15 µL of of the DOTMP solution was added. The pH of the solution was adjusted to 7 to 8 using NaOH. The amount of Gd as a complex was determined to be > 99% by cation exchange chromatography. This formulation has a ligand to metal molar ratio of 1.5.

A Sprague Dawley rat was injected with the above prepared formulation and the biodistribution determined. The rat was allowed to acclimate for five days then injected with 100 μL of the Gd solution described above via a tail vein. The rat weighed between 150 and 200 g at the time of injection. After 2 hours the rat was killed by cervical dislocation. Tissues were taken, weighed and the amount of radioactivity in each tissue was determined by counting in a NaI scintillation counter coupled to a multichannel analyzer. The counts in each tissue were compared to the counts in 100 μL standards in order to determine the percentage of the injected dose in each tissue or organ. The percent of the injected dose in several tissues is given in Table 1. The ligand (DOTMP) to metal (Gd) molar ratio = 1.5.

TABLE 1
% OF INJECTED DOSE (159Gd)
IN VARIOUS TISSUES

| Tissue | Ho-DOTMP (0.32 mg/mL) |
|---|---|
| Bone | 50 |
| Liver | 0.08 |
| Kidney | 0.25 |
| Muscle | N.D.[1] |
| Blood | 0.08 |

[1]N.D. means counts in spleen were below background

Example 4: Preparation of Gd-DOTMP Complex

A 54.3 mg (86.3 μMol) sample of DOTMP, prepared by the procedure of Example B, was dissolved in 4 mL of water by adding 0.049 mL of concentrated $NH_4OH$. This gave a 0.021M solution of DOTMP at pH = 6.33. A 3 mL (64 μMol) portion of this ligand solution was heated to 82°C and treated with 291 μL (58 μMol) of 0.2M gadolinium triacetate solution. The addition of gadolinium was made over a 20 min period in small (20-50 μL) portions. During the addition the pH was maintained greater than 6.0 by the addition of 5 μL portions of concentrated $NH_4OH$. Upon complete addition of all the gadolinium triacetate solution, the pH was adjusted from 6.56 to 7.53 by the addition of 20 μL more of $NH_4OH$. This solution of the complex was cooled to room temperature whereupon the pH was found to be 8.11. Anion exchange HPLC analysis of this solution showed 12% (by UV integration) ligand present and 88% (by UV integration) Gd-DOTMP complex present in the solution. This result compares favorably with the 10% ligand and 90% complex the theoretically could have been present.

Example 5: Biodistribution of Gd-DOTMP Complex in a Rat

A 150 g Sprague Dawley rat was injected with 6-100 μL portions of the Gd-DOTMP, prepared in Example 4, over a 4 day period. This rat was then sacrificed and one of its femur bones was removed and found to weigh 0.690 g. This bone was dissolved in 5 mL of concentrated HCl and analyzed for Gd by inductively coupled plasma spectroscopy. The amount of Gd found in the femur bone was 16.76 μg, which corresponds to 419 μg in the total rat skeleton, assuming the femur is 1/25th of the total skeleton. This corresponds to 25.5% of the injected dose going to the bone. That result compares favorably with similar work done with radioactive tracer $^{153}Sm$ where at these high levels of injected doses about 25-30% of the injected dose localizes in the bone. Thus the behavior of Sm-DOTMP and Gd-DOTMP are very similar *in vivo*.

Other embodiments of the invention will be apparent to those skilled in the art from a consideration of this specification or practice of the invention disclosed herein. It is intended that the specification and examples be considered as exemplary only, with the true scope and spirit of the invention being indicated by the following claims.

## Claims

1. A method of obtaining an magnetic resonance image of an animal which comprises administering to the animal a composition comprising a calcific site seeking complex of:-
(1) a macrocyclic aminophosphonic acid of the formula

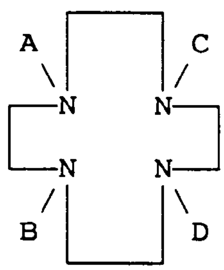

(I)

wherein: A, B, C and D are each independently hydrogen, a hydrocarbyl group having from 1 to 8 carbon atoms, or a moiety of the formula:-

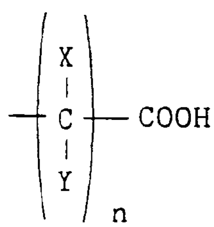 , 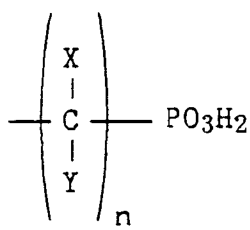 , or 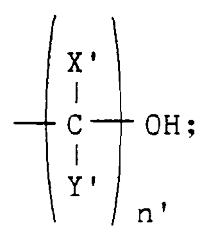

or a physiologically acceptable salt of such a moiety, wherein each X and each Y independently is hydrogen or a hydroxyl, carboxyl, phosphonic or hydrocarbyl group having from 1 to 8 carbon atoms, each X′ and Y′ independently is hydrogen, a methyl or an ethyl group; n′ is 2 or 3, with the proviso that at least two of said nitrogen substituents is A, B, C and D a phosphorus-containing group; and (2) at least one paramagnetic nuclide; wherein the molar ratio of (1) to (2) is at least 1:1.

2. A method of Claim 1 wherein the macrocyclic aminophosphonic acid is 1,4,7,10-tetraazacyclododecane1,4,7,10-tetramethylenephosphonic acid.

3. A method of Claim 1 or Claim 2 wherein the paramagnetic nuclide is $Gd^{+3}$, $Fe^{+3}$, $Mn^{+2}$ or $Mn^{+3}$.

4. A method of Claim 3 wherein the paramagnetic nuclide is $Gd^{+3}$.

5. A method as claimed in any one of Claims 1 to 4, wherein the composition also comprises a pharmaceutically-acceptable carrier, excipient or vehicle.

6. A method as claimed in Claim 5 wherein the complex comprises 1,4,7,10-tetraazacyclododecane-1,4,7,10-tetramethylenephosphonic acid with $Gd^{+3}$.

7. The use in the preparation of a composition for magnetic resonance imaging of an animal, of a complex of
(1) a macrocyclic aminophosphonic acid of the formula

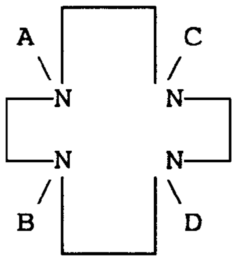

(I)

wherein: A, B, C and D are as defined in Claim 1, with
(2) at least one paramagnetic nuclide, wherein the molar ratio of (1) to (2) is at least 1:1.

8. The use as claimed in Claim 7 wherein the macrocyclic aminophosphonic acid is 1,4,7,10-tetraazacyclododecane-1,4,7,10-tetramethylenephosphonic acid.

9. The use as claimed in Claim 7 or Claim 8, wherein the paramagnetic nuclide is $Gd^{+3}$, $Fe^{+3}$, $Mn^{+2}$ or $Mn^{+3}$.

10. the use as claimed in any one of Claims 7 to 10, wherein the composition also comprises a pharmaceutically-acceptable carrier, excipient or vehicle.

11. A method as claimed in any one of Claims 1 to 6, or the use as claimed in Claim 7, wherein molar ratio of (1) to (2) is from 1:1 to 5:1.

12. A method as claimed in any one of Claims 1 to 6, or the use as claimed in Claim 7, wherein molar ratio of (1) to (2) is from 1:1 to 3:1.

13. A method as claimed in any one of Claims 1 to 6, or the use as claimed in Claim 7, wherein molar ratio of (1) to (2) is about 1:1.

European Patent Office

# PARTIAL EUROPEAN SEARCH REPORT

which under Rule 45 of the European Patent Convention shall be considered, for the purposes of subsequent proceedings, as the European search report

Application Number: EP 91 30 5484

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,Y | FR-A-2 539 996 (SCHERING AG)<br>* Page 1, line 22 - page 5, line 5; page 7, lines 11-26; example 11 *<br>--- | 7-13 | A 61 K 49/00 |
| D,Y<br>P | EP-A-0 375 376 (THE DOW CHEMICAL CO.)<br>* Claims *<br>--- | 7-13 | |
| X,Y | WO-A-8 901 476 (CELLTECH LTD)<br>* Claims 1-3,7,8 *<br>--- | 7-13 | |
| X | JOURNAL OF MAGNETIC RESONANCE, vol. 76, 1988, pages 528-533, Academic Press, Inc.; A. D. SHERRY et al.: "Dy(DOTP)5-: A new stable 23Na shift reagent"<br>* Page 528, paragraph 1 - page 529, paragraph 1 *<br>--- | 7,8,10-13 | |
| Y | IDEM.<br>--- -/- | 7-13 | |

TECHNICAL FIELDS SEARCHED (Int. Cl.5)

A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely : 7-10

Claims searched incompletely : 11-13

Claims not searched : 1-6

Reason for the limitation of the search:

Method for treatment of the human or animal body by surgery or therapy (see article 52(4) of the European Patent Convention).

See sheet -C-

| Place of search | Date of completion of the search | Examiner |
|---|---|---|
| THE HAGUE | 15-10-1991 | SITCH W.D.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0407)

European Patent Office

# PARTIAL EUROPEAN SEARCH REPORT

Application Number
EP 91 30 5484

| DOCUMENTS CONSIDERED TO BE RELEVANT | | | CLASSIFICATION OF THE APPLICATION (Int. Cl.5) |
|---|---|---|---|
| Category | Citation of document with indication, where appropriate, of relevant passages | Relevant to claim | |
| Y | INTERNATIONAL JOURNAL OF RADIATION/APPLICATIONS AND INSTRUMENTATION PART B, vol. 15, no. 1, 1988, Pergamon Journals Ltd, Marsh Barton, Exeter, GB; J. F. DESREUX et al.: "Highly stable lanthanide macrocylic complexes: in search of new contrast agents for NMR imaging" * The whole article * --- | 7-13 | |
| Y | WO-A-9 002 571 (COMPAGNIE ORIS INDUSTRIE S.A.) * Claim 1 * --- | 7-13 | |
| P,X | EP-A-0 382 582 (CELLTECH LTD) * Exampe 4; claims * ----- | 7-13 | TECHNICAL FIELDS SEARCHED (Int. Cl.5) |

EPO FORM 1503 03.82 (P0410)

EP 91 30 5484 -C-

Claims searchable completely 2,6,8:

Claims searchable incompletely 1,3-5,7,9-13:

There is no definition of "n" given in the claims. From interpretation of the examples this has been assumed to be equal to 1.
The claims referred to in claim 10 have been assumed to be claims 7-9, and not 7-10 as given, see EPC Article 84.